# EUROPEAN PATENT APPLICATION

(11) **EP 1 724 345 A1**
(43) Date of publication of application: **22.11.2006**
(21) Application number: 05719183.5
(22) Date of filing: 16.02.2005
(51) Int. Cl.: C12N 15/09, C12Q 1/68

(54) **METHOD OF DETECTING AND IDENTIFYING LACTIC ACID BACTERIUM**

(30) Priority: 17.02.2004 JP 2004040381
(71) Applicant: Sapporo Breweries Limited, Tokyo 150-8522 (JP)
(72) Inventor: NAKAKITA, Yasukazu, c/o Sapporo Breweries Limited, Yaizu-shi, Shizuoka 425-0013 (JP); TSUCHIYA, Youichi, c/o Sapporo Breweries Limited, Yaizu-shi, Shizuoka 425-0013 (JP)
(74) Representative: Vossius & Partner
(86) International application number: PCT/JP2005/002331
(87) International publication number: WO 2005/078086

(57) **Abstract**

There are provided a method of detecting potentially beer-spoiling lactic acid bacteria, and a method of simultaneously detecting and distinguishing different lactic acid bacteria including such bacteria.

## Description

### Technical Field

The present invention relates to a method of detecting and distinguishing lactic acid bacteria.

### Background Art

As a greater proportion of beer produced in recent years is draft beer, a new awareness has risen regarding the freshness of beer. Under such circumstances, beer brewing companies are being forced to rapidly and accurately identify microbial contaminants that spoil beer (beer-spoilage microorganisms) in order to dramatically shorten the time from beer production to shipping.

Lactic acid bacteria are among the most commonly contaminating beer-spoilage microorganisms. Various detection methods for rapid detection of lactic acid bacteria are already known such as the PCR method (Polymerase Chain Reaction) and FISH method *(Fluorescence in situ* Hybridization).

[Patent document 1] Japanese Unexamined Patent Publication No. 5-15400
[Patent document 2] Japanese Unexamined Patent Publication No. 6-141899
[Patent document 3] Japanese Unexamined Patent Publication No. 7-289295
[Patent document 4] Japanese Unexamined Patent Publication No. 10-210980 [Patent document 5] Japanese Unexamined Patent Publication No. 14-034578

### Disclosure of the Invention

### Problems to be Solved by the Invention

However, the present inventors have discovered novel beer-spoiling lactic acid bacteria and have found that the bacteria cannot be detected by the aforementioned prior art methods.

It is therefore an object of the present invention to provide a method for detecting potentially beer-spoiling lactic acid bacteria, which have not been detectable by prior art methods. It is another object of the present invention to provide a method for simultaneously detecting and distinguishing different types of beer-spoilage lactic acid bacteria including said bacteria.

### Means for Solving the Problems

In order to achieve the aforestated objects, the present invention provides polynucleotides comprising all or portions of the nucleotide sequences as set forth in SEQ ID NOS: 1-5.

The present invention also provides a primer set for detection of *Lactbacillus hexosus* comprising an oligonucleotide consisting of the nucleotide sequence as set forth in SEQ ID NO: 6 and an oligonucleotide consisting of the nucleotide sequence as set forth in SEQ ID NO: 7, as well as a method for detecting *Lactbacillus hexosus* by gene amplification characterized by comprising a step of amplifying a nucleic acid fragment using the aforementioned primer set and a step of detecting the obtained nucleic acid fragment.

The present invention also provides a primer set for detection of *Lactbacillus pseudocollinoides* comprising an oligonucleotide consisting of the nucleotide sequence as set forth in SEQ ID NO: 7 and an oligonucleotide consisting of the nucleotide sequence as set forth in SEQ ID NO: 8, as well as a method for detecting *Lactbacillus pseudocollinoides* by gene amplification characterized by comprising a step of amplifying a nucleic acid fragment using the aforementioned primer set and a step of detecting the obtained nucleic acid fragment.

The present invention also provides a primer set for detection of *Pediococcus damnosus* comprising an oligonucleotide consisting of the nucleotide sequence as set forth in SEQ ID NO: 9 and an oligonucleotide consisting of the nucleotide sequence as set forth in SEQ ID NO: 10, as well as a method for detecting *Pediococcus damnosus* by gene amplification characterized by comprising a step of amplifying a nucleic acid fragment using the aforementioned primer set and a step of detecting the obtained nucleic acid fragment.

The present invention further provides a primer set for detecting and distinguishing lactic acid bacteria comprising oligonucleotides consisting of the nucleotide sequences as set forth in SEQ ID NOS: 30 and 11-14, a probe set for detecting and distinguishing lactic acid bacteria comprising oligonucleotides consisting of the nucleotide sequences as set forth in SEQ ID NO: 15-19, a kit for detecting and distinguishing lactic acid bacteria characterized by comprising the aforementioned primer set and probe set, and a method for detecting and distinguishing lactic acid bacteria characterized by comprising a step of amplifying a nucleic acid fragment using the aforementioned primer set and a step of measuring the melting temperature for a hybrid of the obtained nucleic acid fragment and the probe set.

The present inventors have succeeded in isolating the potentially beer-spoiling lactic acid bacteria *L. hexosus* and *L. pseudocollinoides,* which are not detectable by known methods, and have shown that the nucleotide sequences of the 16S ribosomal RNA genes (16S rRNA genes) of *L. hexosus* SBC8050 (unapproved name) and *L. pseudocollinoides* SBC8057 (unapproved name) are the nucleotide sequences as set forth in SEQ ID NOS: 1 and 3, respectively, and that portions of the nucleotide sequences of the *gyrB* genes are the nucleotide sequences as set forth in SEQ ID NOS: 2 and 4, respectively. The present inventors have also shown that a portion of the nucleotide sequence of the *gyrB* gene of *Pediococcus damnosus,* a lactic acid bacterium which is capable of growing in beer, is the nucleotide sequence as set forth in SEQ ID NO: 5. The *L. hexosus* SBC8050 and *L. pseudocollinoides* SBC8057 strains have been deposited at the National Institute of Advanced Industrial Science and Technology, International Patent Organism Depositary (IPOD) (Tsukuba Central 6, 1-1, Higashi 1-chome, Tsukuba-shi, Ibaraki-ken, Japan, 305-8566) on October 21, 2003 as FERM BP-08529 and FERM BP-08530, respectively.

By using a primer set for detection of *L. hexosus,* a primer set for detection of *L. pseudocollinoides* or a primer set for detection of *P. damnosus,* it is possible to specifically amplify the *gyrB* gene of *L. hexosus, L. pseudocollinoides* or *P. damnosus,* respectively and thereby detect *L. hexosus, L. pseudocollinoides* or *P. damnosus,* respectively.

Also, by using a primer set for detecting and distinguishing lactic acid bacteria it is possible to amplify the 16S rRNA or *gyrB* gene of different lactic acid bacteria. Moreover, by measuring the melting temperature for a hybrid of the obtained nucleic acid fragment and the probe set for detecting and distinguishing lactic acid bacteria, it is possible to detect and distinguish lactic acid bacteria based on differences in melting temperature, since the melting temperature differs according to the type of beer-spoiling bacterium from which the nucleic acid fragment is derived.

### Effect of the Invention

There are provided a method of detecting potentially beer-spoiling lactic acid bacteria that have not been detectable by prior art methods, and a method of simultaneously detecting and distinguishing different lactic acid bacteria including said bacteria.

### Brief Explanation of the Drawings

Fig. 1 shows melting curves for (a) *L. brevis* SBC8003 and (b) *L. hexosus* SBC8050 at 640 nm.
Fig. 2 shows melting curves for (c) *L. collinoides* JCM1123, (d) *P. damnosus* JCM5886 and (e) *L. pseudocollinoides* SBC8057 at 710 nm.

### Best Mode for Carrying Out the Invention

Preferred embodiments of the present invention will now be described in detail.

### <Polynucleotides>

The polynucleotides of the present invention will be explained first. A polynucleotide according to the present invention is characterized by comprising all or a portion of the nucleotide sequences as set forth in SEQ ID NOS: 1-5, but also included are polynucleotides comprising all or a portion of the sequences complementary to the nucleotide sequences as set forth in SEQ ID NOS: 1-5. Here, SEQ ID NOS: 1 and 3 represent the nucleotide sequences for the 16S rRNA genes of *L. hexosus* and *L. pseudocollinoides,* respectively. Also, SEQ ID NOS: 2, 4 and 5 represent the nucleotide sequences for the *gyrB* genes of *L. hexosus, L. pseudocollinoides* and *P. damnosus,* respectively. As described hereunder, the polynucleotides of the present invention are useful for detection of the aforementioned lactic acid bacteria, and can be used as detection primers for the lactic acid bacteria, as nucleic acid fragments that are amplified by the primers and as detection probes. The polynucleotides of the invention may also be chemically modified with fluorescent substances or the like.

When a polynucleotide of the present invention is used as a detection primer or detection probe for lactic acid bacteria, it is preferably an oligonucleotide with a nucleotide length of 10-30 (preferably 15-25). Primers may be designed easily by a person skilled in the art, if necessary utilizing primer design assistant software.

According to the present invention, the terms "polynucleotide" and "oligonucleotide" encompass DNA, RNA and PNA (peptide nucleic acid). Polynucleotides and oligonucleotides of the invention may be synthesized by publicly known methods such as, for example, the phosphoramidite method.

### <Detection of L. hexosus>

A primer set for detection of *L. hexosus* and a detection method for *L. hexosus* using the primer set according to the present invention will now be explained. The primer set for detection of *L. hexosus* according to the present invention is characterized by comprising an oligonucleotide consisting of the nucleotide sequence as set forth in SEQ ID NO: 6 and an oligonucleotide consisting of the nucleotide sequence as set forth in SEQ ID NO: 7, and since both oligonucleotides have nucleotide sequences specific to the *gyrB* gene of *L. hexosus,* they allow specific amplification of the *gyrB* of *L. hexosus* for specific detection of *L. hexosus.*

For detection of *L. hexosus* by the detection method of the present invention, first nucleic acid is extracted from a test sample (for example, a malt beverage such as beer or low-malt beer (happoshu)). The nucleic acid extraction may be carried out using a method known in the field, and specifically DNA may be extracted by, for example, a method involving phenol extraction and ethanol precipitation or a method employing glass beads, while RNA may be extracted by an AGPC method or guanidine/cesium chloride ultracentrifugation.

The obtained nucleic acid is then used as template for amplification of a nucleic acid fragment using the aforementioned primer set. The amplification method used may be an amplification method which is known in the field, and particularly PCR or RT-PCR is preferred. In the case of PCR, a nucleic acid fragment consisting of the nucleotide sequence of the portion of the *gyrB* gene between the primer set is amplified by using DNA polymerase and the extracted DNA as template. In the PCR, a cycle consisting of denaturation, annealing and complementary chain synthesis is repeated to yield a nucleic acid fragment (double-stranded DNA), and the optimum PCR conditions such as the temperature and time for each step and the number of cycles may be easily determined by a person skilled in the art. In RT-PCR, extracted RNA is used as template for synthesis of cDNA with reverse transcriptase, and the obtained cDNA is then used as template for PCR.

The amplified nucleic acid fragment is detected next. Specifically, it is determined whether or not the amplified nucleic acid fragment is specific to *L. hexosus.* The detection may be carried out by a method known in the field, and for example, it may be carried out by hybridization using a probe that specifically hybridizes to *L. hexosus.*

### <Detection of L. pseudocollinoides>

A primer set for detection of *L. pseudocollinoides* and a method for detection of *L. pseudocollinoides* using the primer set according to the present invention will now be explained. The primer set for detection of *L. pseudocollinoides* according to the present invention is characterized by comprising an oligonucleotide consisting of the nucleotide sequence as set forth in SEQ ID NO: 7 and an oligonucleotide consisting of the nucleotide sequence as set forth in SEQ ID NO: 8, and since both oligonucleotides have nucleotide sequences specific to the *gyrB* gene of *L. pseudocollinoides,* they allow specific amplification of the *gyrB* of *L. pseudocollinoides* for specific detection of *L. pseudocollinoides.*

The method of detecting *L. pseudocollinoides* may be carried out in the same manner as the method of detecting *L. hexosus* described above.

### <Detection of P. damnosus>

A primer set for detection of *P. damnosus* and a method of detecting *P. damnosus* using the primer set according to the invention will now be explained. The primer set for detection of *P. damnosus* according to the present invention is characterized by comprising an oligonucleotide consisting of the nucleotide sequence as set forth in SEQ ID NO: 9 and an oligonucleotide consisting of the nucleotide sequence as set forth in SEQ ID NO: 10, and since both oligonucleotides have nucleotide sequences specific to the *gyrB* gene, they allow specific amplification of the *gyrB* of *P. damnosus* for specific detection of *P. damnosus.*

The method of detecting *P. damnosus* may be carried out in the same manner as the method of detecting *L. hexosus* described above.

### <Detection and distinction of lactic acid bacteria>

A primer set, probe set and kit for detecting and distinguishing lactic acid bacteria and a method for detecting and distinguishing lactic acid bacteria using them will now be explained.

The primer set for detecting and distinguishing lactic acid bacteria according to the invention is characterized by comprising oligonucleotides consisting of the nucleotide sequences as set forth in SEQ ID NOS: 30 and 11-14. The oligonucleotide consisting of the nucleotide sequence as set forth in SEQ ID NO: 30 is a universal primer for the 16S rRNA gene. By using the primer set of the present invention it is possible to amplify nucleic acid fragments of different lactic acid bacteria.

The probe set for detecting and distinguishing lactic acid bacteria according to the present invention is characterized by comprising oligonucleotides consisting of the nucleotide sequences as set forth in SEQ ID NOS: 15-18. As described below, the probe set can detect nucleic acid fragments of different lactic acid bacteria, so that lactic acid bacteria can be detected and distinguished.

The kit for detecting and distinguishing lactic acid bacteria according to the present invention is characterized by comprising the aforementioned primer set and probe set. The kit may also contain a reaction buffer, dNTP mixture and enzymes, as well as a DNA extraction reagent.

In order to detect and distinguish lactic acid bacteria by the detecting and distinguishing method of the present invention, first nucleic acid is extracted from a sample (for example, a malt beverage such as beer or low-malt beer (happoshu)). The nucleic acid extraction may be carried out in the same manner as described above.

The obtained nucleic acid is then used as template for amplification of a nucleic acid fragment using the aforementioned primer set. The amplification may be carried out by the same method as described above, and PCR or RT-PCR is preferred.

Next, the obtained nucleic acid fragment and the probe set are hybridized and the hybrid melting temperature is measured. The principle for measurement of the melting temperature is explained below. The oligonucleotides consisting of the nucleotide sequences as set forth in SEQ ID NOS: 15-16 and 17-18 are labeled at their respective 5' ends with the fluorescent substances LC Red640 and LC Red705 (hereinafter these will be referred to as "Red640 probe" and "Red705 probe", respectively). While the oligonucleotide consisting of the nucleotide sequence as set forth in SEQ ID NO: 19 is labeled at the 3' end with FITC (hereinafter, this will be referred to as "FITC probe"). Each probe is designed to hybridize with the nucleic acid fragment of the beer-spoilage microorganism in a manner with the 3' end of the FITC probe and the 5' ends of the Red640 probe and Red705 probe in proximity. When light having the excitation wavelength for FITC is irradiated on the hybrid with both the FITC probe and the Red640 probe (or Red705 probe) hybridized to the nucleic acid fragment, FRET (Fluorescence Resonance Energy Transfer) occurs and light of the fluorescent wavelength of Red640 (or Red705) is observed. When the temperature is raised in this state, the FITC probe and/or Red640 probe (or Red705 probe) melts and separates from the nucleic acid fragment, resulting in cessation of FRET and reduction in the fluorescent intensity of Red640 (or Red705). The fluorescent intensity is measured at each temperature, and by plotting temperature on the horizontal axis and fluorescent intensity (including changing ratio) on the vertical axis, a melting curve is obtained. Analysis of the melting curve obtained in this manner permits the hybrid melting temperature to be determined.

The FITC probe and/or Red640 probe (or Red705 probe) are set so that the degree of mismatch with the nucleic acid fragment differs depending on the type of lactic acid bacteria. Thus, since the melting curve and melting temperature exhibited by the hybrid will differ depending on the type of lactic acid bacteria, the type of lactic acid bacteria can be distinguished based on the difference. Specifically, the melting temperatures exhibited are approximately 60°C for *L. brevis,* approximately 56°C and approximately 63°C for *L. hexosus* SBC8050, approximately 62°C for *P. damnosus* JCM5886, approximately 64°C for *L. pseudocollinoides* SBC8057 and approximately 58°C for *L. collinoidés* JCM1123. Comparing these melting temperatures with the melting temperature of the sample will allow the lactic acid bacteria in the sample to be detected and distinguished.

Since the probe set of the present invention can be mixed with the reaction solution used for amplification of the nucleic acid fragment, the melting temperature can be measured immediately after completion of the nucleic acid fragment amplification reaction. Thus, the detecting and distinguishing method for lactic acid bacteria according to the present invention has the advantage of allowing the step of amplifying the nucleic acid fragment and the step of measuring the melting temperature to be carried out continuously in the same tube or capillary.

### Examples

The present invention will now be explained in greater detail using examples, with the understanding that the invention is not limited to the examples.

### (Example 1) Spoilage of beer by inoculation of L. hexosus and L. pseudocollinoides

Strains *L. hexosus* SBC8050 and *L. pseudocollinoides* SBC8057 were each grown on MRS agar medium (Becton Dickinson). One loopful of each bacterial strain was inoculated into bottled all malt beer (pH 4.5, bitterness value: 30, alcohol content: 5%, volume: 350 ml), and then the beer bottle was capped and culturing was carried out for about 1 month at 30°C, resulting in spoilage of the beer.

Table 1 shows the concentration of organic acids in the spoiled beer after one month of culturing (as a percentage (%) with respect to normal beer). The spoiled beer into which *L. hexosus* SBC8050 had been inoculated had a lactic acid concentration of approximately 3 times that of normal beer. This demonstrated that *L. hexosus* is a homofermenting lactic acid bacterium. On the other hand, the spoiled beer into which *L. pseudocollinoides* SBC8057 had been inoculated had a lactic acid concentration of approximately 4 times, and an acetic acid concentration of approximately 2 times, compared to normal beer. This demonstrated that *L. pseudocollinoides* is a heterofermenting lactic acid bacterium.

**[Table 1]**

| Organic acid | *L. hexosus* SBC8050 | *L. pseudocollinoides* SBC8057 |
|---|---|---|
| Malic acid | 17 | 99 |
| Lactic acid | 326 | 410 |
| Acetic acid | 115 | 191 |
| Succinic acid | 95 | 94 |

### (Example 2) Sequencing of 16S rRNA gene and gyrB gene. (Preparation of genomic DNA)

Strains *L. hexosus* SBC8050 and *L. pseudocollinoides* SBC8057 were each inoculated into MRS agar medium and anaerobically cultured at 30°C for 7-14 days. The anaerobic culturing was carried out using an anaerobic culturing apparatus by Tabai Espec Corp., under conditions of N₂:H₂:CO₂ = 90:5:5. DNA was extracted from the anaerobic cultured bacterial strains using DNA extract reagent PrepMan Ultra (Applied Biosystems Japan, Ltd.).

### (Amplification and analysis of 16S rRNA gene)

The DNA extract prepared by the aforementioned method was used for sequencing of the 16S rRNA gene of each bacterial strain using a MicroSeq Full Gene 16S rDNA kit (Applied Biosystems Japan, Ltd.).

The sequences of the 16S rRNA genes of strains *L. hexosus* SBC8050 and *L. pseudocollinoides* SBC8057 obtained as a result of sequencing are as set forth in SEQ ID NOS: 1 and 3, respectively. These gene sequences clearly differed from the gene sequences of beer-spoiling lactic acid bacteria that have been reported to date. A GenBank database search was also conducted, but no match was found with any registered gene sequence. Thus, *L. hexosus* and *L. pseudocollinoides* were demonstrated to be novel beer-spoiling lactic acid bacteria.

### (Amplification and analysis of gyrB genes)

To a TaKaRa ExTaq (Takara Shuzo) reaction mixture there were added the aforementioned DNA extract and a primer set (using a set with an oligonucleotide consisting of the nucleotide sequence as set forth in SEQ ID NO: 26 (GYPF: 5'-GGWTAYAARGTWTCWGGTGGT-3') and an oligonucleotide consisting of the nucleotide sequence as set forth in SEQ ID NO: 27 (GYPR: 5'-TCATGYGTWCACCTTCAT-3') for *L. hexosus* and *L. pseudocollinoides,* and a set with an oligonucleotide consisting of the nucleotide sequence as set forth in SEQ ID NO: 28 (GP1-F: 5'-ATTATGNTGCNNGNCAAATNCAA-3') and an oligonucleotide consisting of the nucleotide sequence as set forth in SEQ ID NO: 29 (GP1-R: 5'-ACCACCWGAWACYTTRTAWCC-3') for *Pediococcus damnosus*), and PCR was conducted using a GeneAmp PCR System 9700 (Applied Biosystems Japan). The PCR was conducted with 35 cycles, with each cycle consisting of 30 seconds at 95°C (DNA denaturation), 30 seconds at 55°C (annealing) and 45 seconds at 72°C (DNA extension).

Upon completion of PCR, 5 µL of the reaction mixture was subjected to polyacrylamide gel electrophoresis and the PCR product was detected. The electrophoresed gel was dyed for 10 minutes with an ethidium bromide solution and irradiated with ultraviolet rays, and the DNA bands were confirmed by observation.

The nucleotide sequence of the *gyrB* gene was determined with an ABI PRISM dRhodamine Terminator Cycle Sequencing Ready Reaction Kit and Genetic Analyzer ABI PRISM310 (Applied Biosystems Japan, Ltd.), using the GYPF and GYPR primer set or the GP1-F or GP1-R primer set.

The sequences of the *gyrB* genes of strains *L. hexosus* SBC8050 and *L. pseudocollinoides* SBC8057 obtained as a result of sequencing are as set forth in SEQ ID NOS: 2 and 4, respectively. The sequence of the *gyrB* gene of strain *Pediococcus damnosus* SBC8023 is as set forth in SEQ ID NO: 5. A GenBank database search was also conducted for these gene sequences, but no match was found with any registered gene sequence.

### (Example 3) Detection and distinction of L. hexosus, L. pseudocollinoides and P. damnosus utilizing gyrB genes

Following the same method as in Example 2, DNA was extracted from different potentially beer-spoiling bacterial strains and the obtained DNA extracts were subjected to PCR using the following primer sets. A primer set comprising oligonucleotides consisting of the nucleotide sequences as set forth in SEQ ID NOS: 6 and 7 was used as the primer set for *L. hexosus,* a primer set comprising oligonucleotides consisting of the nucleotide sequences as set forth in SEQ ID NOS: 7 and 8 was used as the primer set for *L. pseudocollinoides* and a primer set comprising oligonucleotides consisting of the nucleotide sequences as set forth in SEQ ID NOS: 9 and 10 was used as the primer set for *P. damnosus.* The PCR reaction conditions were the same as in Example 2, and the amplification products were also confirmed in the same manner as Example 2.

Table 2 shows a summary of amplification products obtained or not obtained from different bacterial strains using each primer set.

**[Table 2]**

| Tested strains | *L. hexosus* primer set | *L. pseudocollinoides* primer set | *P. damnosus* primer set |
|---|---|---|---|
| Lactobacillus hexosus SBC8050 | + | - | - |
| Lactobacillus hexosus SBC8051 | + | - | - |
| Lactobacillus hexosus SBC8817 | + | - | - |
| Lactobacillus hexosus SBC8818 | + | - | - |
| Lactobacillus pseudocollinoides SBC8057 | - | + | - |
| Lactobacillus pseudocollinoides SBC8058 | - | + | - |
| Lactobacillus pseudocollinoides SBC8063 | - | + | - |
| Lactobacillus pseudocollinoides SBC8064 | - | + | - |
| Lactobacillus coryniformis subsp. toruens JCM1166 | - | - | - |
| Lactobacillus coryniformis subsp. coryniformis JCM1164 | - | - | - |
| Lactobacillus collinoides JCM1123 | - | - | - |
| Lactobacillus lindneri VTT-E-89362 | - | - | - |
| Lactobacillus sp. SBC8021 | - | - | - |
| Lactobacillus sp. SBC8019 | - | - | - |
| Lactobacillus brevis SBC8003 | - | - | - |
| Lactobacillus plantarum JCM1142 | - | - | - |
| Lactobacillus malefementans JCM1167 | - | - | - |
| Lactobacillus buchneri JCM1115 | - | - | - |
| Lactobacillus paracasei JCM1171 | - | - | - |
| Pediococcus damnosus JCM5886 | - | - | + |
| Pediococcus damnosus SBC8023 | - | - | + |
| Pediococcus damnosus SBC8024 | - | - | + |
| Pediococcus acidilactici ID152-3 | - | - | - |

| | | | |
|---|---|---|---|
| +: Amplification product present -: Amplification product absent JCM: Japan Collection of Microorganisms, Saitama, Japan VTT: Valtion Teknillinen Tutkimuskeskus, Finland SBC, ID: Isolated by Sapporo Breweries Ltd. | | | |

As seen by the results in Table 2, an amplification product (322 bp) was confirmed only with bacterial strains belonging to *L. hexosus* when using the primer set for *L. hexosus,* an amplification product (362 bp) was confirmed only with bacterial strains belonging to *L. pseudocollinoides* when using the primer set for *L. pseudocollinoides,* and an amplification product (194 bp) was confirmed only with bacterial strains belonging to *P. damnosus* when using the primer set for *P. damnosus.* This demonstrated that these primer sets can be used for specific detection of *L. hexosus, L. pseudocollinoides* and *P. damnosus.* Some of the strains used (isolated by Sapporo Breweries Ltd.) were identified based on their 16S rRNA gene sequences by the same method as in Example 2.

### (Example 4) Detection and distinction of lactic acid bacteria using real-time PCR

DNA extracts from different bacterial strains prepared by the same method as in Example 2 were subjected to PCR with the reaction reagent composition shown in Table 3, using the following primers and probes. As primers there were used an oligonucleotide consisting of the nucleotide sequence as set forth in SEQ ID NO: 30 (a universal primer for the16S rRNA gene, 5'-TGGAGAGTTTGATCCTGGCTC-3') and oligonucleotides consisting of the nucleotide sequences as set forth in SEQ ID NOS: 11-14. As probes there were used oligonucleotides consisting of the nucleotide sequences as set forth in SEQ ID NOS: 15 and 16 (phosphorylated at the 3'-end and labeled with LC Red640 at the 5'-end), oligonucleotides consisting of the nucleotide sequences as set forth in SEQ ID NOS: 17 and 18 (phosphorylated at the 3'-end and labeled with LC Red705 at the 5'-end) and an oligonucleotide consisting of the nucleotide sequence as set forth in SEQ ID NO: 19 (labeled with FITC at the 3'-end).

**[Table 3]**

| Reagent | Volume |
|---|---|
| LightCycler-FastStart DNA Master Hybridization Probes* | 2.0 µL |
| Primer (10 µM) | (each) 1.0 µL |
| Probe (10 µM) | (each) 0.4 µL |
| MgCl₂ (25 mM) | 1.6 µL |
| Sterilized water | 7.5 µL |
| DNA extract | 0.5 µL |
| Total | 20.0 µL |

| | |
|---|---|
| *: Roche Diagnostics K.K. | |

The PCR was conducted by using a LightCycler Quick System 330 (Roche Diagnostics, KK.) as the reaction apparatus by means of 10 minutes of treatment at 95°C followed by repetition of 40 cycles with each cycle consisting of 15 seconds at 95°C, 5 seconds at 50°C and 20 seconds at 72°C.

Upon completion of the PCR the temperature was raised to 95°C; and after immediate cooling to 40°C, the temperature was held for 15 seconds and the temperature was raised again to 95°C at a rate of 20°C/sec. During heating, the fluorescent intensity at 640 nm and 710 nm was measured at every 0.2°C, the negative of the first derivative of the value (-dF/dT) was plotted, and the resulting peak was used to determine the melting temperature.

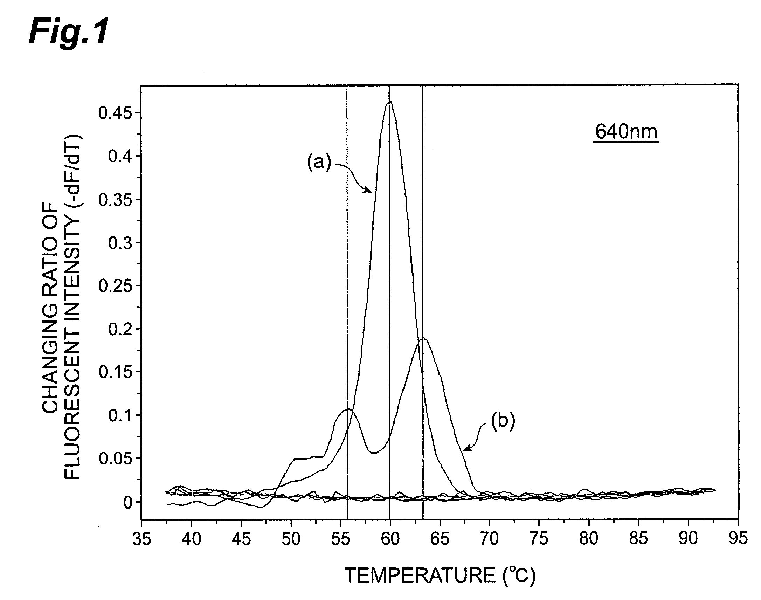

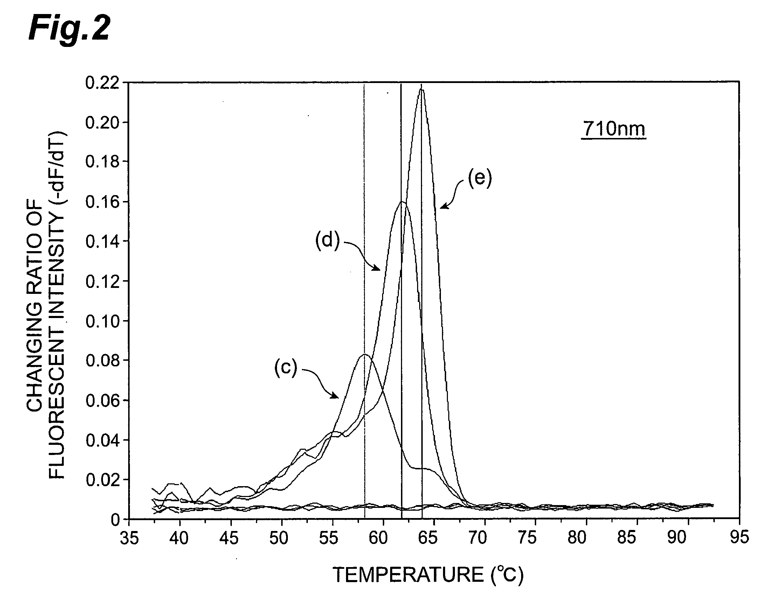

Figs. 1 and 2 show melting curves representing the relationship between changing ratio of fluorescent intensity (-dF/dT) and temperature (°C). Fig. 1 shows melting curves for (a) *L. brevis* SBC8003 and (b) *L. hexosus* SBC8050 at 640 nm, and Fig. 2 shows melting curves for (c) *L. collinoides* JCM1123, (d) *P. damnosus* JCM5886 and (e) *L. pseudocollinoides* SBC8057 at 710 nm.

As is clear from the results shown in Figs. 1 and 2, peaks were obtained representing melting temperatures of about 60°C at 640 nm for *L. brevis* SBC8003, of about 56°C and 63°C at 640 nm for *L. hexosus* SBC8050, of about 62°C at 710 nm for *P. damnosus* JCM5886 and of about 64°C at 710 nm for *L. pseudocollinoides* SBC8057. A peak representing a melting temperature of about 58°C at 710 nm was also observed for *L. collinoides* JCM1123, which is not a potentially beer-spoiling lactic acid bacterium, but since it did not overlap with any of the peaks of the aforementioned potentially beer-spoiling lactic acid bacteria, it was possible to distinguish the strains. The strains used in this example were the same as shown in Table 2, but strains other than those mentioned above exhibited no peaks in the melting curve.

Thus, it was demonstrated that the aforementioned primers and probes can be used to detect and distinguish all potentially beer-spoiling lactic acid bacteria in a single type of reaction mixture.

### (Example 5) Separate detection and distinction of lactic acid bacteria using real-time PCR

The lactic acid bacteria detected and distinguished in Example 4 were examined under conditions for confirmatory testing. As a result, confirmatory testing could be accomplished by carrying out the same method as in Example 4 using the suitable primers and probes listed in Table 4.

**[Table 4]**

| Strain | Primer | Probe | Melting temperature |
|---|---|---|---|
| *L. hexosus* | SEQ ID No: 6, 8, GYPR | SEQ ID No: 20*, 21** | approx. 57°C |
| *L. pseudocollinoides* | SEQ ID No: 6, 8, GYPR | SEQ ID No: 20*, 21** | approx. 49°C |
| *L. brevis* | SEQ ID No: 25, GYPR | SEQ ID No: 22*, 23*** | approx. 62°C |
| *L. brevis* | SEQ ID No: 11, 24 | SEQ ID No: 15***, 19* | approx. 60°C |

| | | | |
|---|---|---|---|
| *: Labeled with FITC at 3'-end **: Phosphorylated at 3'-end, labeled with LC Red705 at 5'-end ***: Phosphorylated at 3'-end, labeled with LC Red640 at 5'-end | | | |

### Industrial Applicability

The present invention allows different beer-spoilage bacteria to be detected and distinguished simultaneously, and can therefore be used for quality control of beer.

## Claims

1. A polynucleotide comprising all or a portion of any of the nucleotide sequences as set forth in SEQ ID NOS: 1-5.

2. A primer set for detecting *Lactbacillus hexosus* comprising an oligonucleotide consisting of the nucleotide sequence as set forth in SEQ ID NO: 6 and an oligonucleotide consisting of the nucleotide sequence as set forth in SEQ ID NO: 7.

3. A primer set for detecting *Lactbacillus pseudocollinoides* comprising an oligonucleotide consisting of the nucleotide sequence as set forth in SEQ ID NO: 7 and an oligonucleotide consisting of the nucleotide sequence as set forth in SEQ ID NO: 8.

4. A primer set for detecting *Pediococcus damnosus* comprising an oligonucleotide consisting of the nucleotide sequence as set forth in SEQ ID NO: 9 and an oligonucleotide consisting of the nucleotide sequence as set forth in SEQ ID NO: 10.

5. A primer set for detecting and distinguishing lactic acid bacteria, comprising an oligonucleotide consisting of the nucleotide sequence as set forth in SEQ ID NO: 30, an oligonucleotide consisting of the nucleotide sequence as set forth in SEQ ID NO: 11, an oligonucleotide consisting of the nucleotide sequence as set forth in SEQ ID NO: 12, an oligonucleotide consisting of the nucleotide sequence as set forth in SEQ ID NO: 13 and an oligonucleotide consisting of the nucleotide sequence as set forth in SEQ ID NO: 14.

6. A probe set for detecting and distinguishing lactic acid bacteria, comprising an oligonucleotide consisting of the nucleotide sequence as set forth in SEQ ID NO: 15, an oligonucleotide consisting of the nucleotide sequence as set forth in SEQ ID NO: 16, an oligonucleotide consisting of the nucleotide sequence as set forth in SEQ ID NO: 17, an oligonucleotide consisting of the nucleotide sequence as set forth in SEQ ID NO: 18 and an oligonucleotide consisting of the nucleotide sequence as set forth in SEQ ID NO: 19.

7. A kit for detecting and distinguishing lactic acid bacteria, comprising a primer set according to claim 5 and a probe set according to claim 6.

8. A method for detecting *Lactbacillus hexosus* by gene amplification comprising a step of amplifying a nucleic acid fragment using a primer set according to claim 2 and a step of detecting the obtained nucleic acid fragment.

9. A method for detecting *Lactbacillus pseudocollinoides* by gene amplification comprising a step of amplifying a nucleic acid fragment using a primer set according to claim 3 and a step of detecting the obtained nucleic acid fragment.

10. A method for detecting *Pediococcus damnosus* by gene amplification comprising a step of amplifying a nucleic acid fragment using a primer set according to claim 4 and a step of detecting the obtained nucleic acid fragment.

11. A method for detecting and distinguishing lactic acid bacteria comprising a step of amplifying a nucleic acid fragment using a primer set according to claim 5 and a step of measuring the melting temperature of a hybrid between the obtained nucleic acid fragment and a probe set according to claim 6.
